# EUROPEAN PATENT SPECIFICATION

(11) **EP 3 357 908 B1**
(45) Date of publication and mention of the grant of the patent: **09.12.2020**
(21) Application number: 16851632.6
(22) Date of filing: 28.09.2016
(51) Int. Cl.: C07D 211/22, C07B 41/06, C07B 61/00, C07C 247/10, C07H 17/08, B01J 19/00

(54) **METHOD FOR PRODUCING ALDEHYDE COMPOUND OR KETONE COMPOUND**
VERFAHREN ZUR HERSTELLUNG EINER ALDEHYDVERBINDUNG ODER KETONVERBINDUNG
PROCÉDÉ DE PRODUCTION D'UN COMPOSÉ ALDÉHYDE OU D'UN COMPOSÉ CÉTONE

(30) Priority: 30.09.2015 JP 2015195376
(43) Date of publication of application: 08.08.2018
(73) Proprietor: FUJIFILM Wako Pure Chemical Corporation, Osaka-shi, Osaka (JP)
(72) Inventor: KEMMOKU, Akira, Hiratsuka-shi Kanagawa 254-0016 (JP); HAGA, Yayoi, Hiratsuka-shi Kanagawa 254-0016 (JP); KAWAMOTO, Fumio, Hiratsuka-shi Kanagawa 254-0016 (JP)
(74) Representative: Forstmeyer, Dietmar
(86) International application number: PCT/JP2016/078624
(87) International publication number: WO 2017/057461

(56) References cited:
- WO-A1-2005/073155
- JP-A- 2009 542 733
- RENE BECKER ET AL: "Flow chemistry today: Practical approaches for optimisation and scale-up", RESEARCHGATE, vol. 29, no. 3, 1 January 2011 (2011-01-01), XP055568680,
- VAN DER LINDEN JACOBUS J. M. et al.: "Investigation of the Moffatt-Swern Oxidation in a Continuous Flow Microreactor System", Organic Process Research & Development, vol. 12, no. 5, 2008, pages 911-920, XP055371074, ISSN: 1083-6160
- KAWAGUCHI TATSUYA et al.: "Room-Temperature Swern Oxidations by Using a Microscale Flow System", Angewandte Chemie International Edition, vol. 44, no. 16, 2005, pages 2413-2416, XP001235003, ISSN: 1521-3773, DOI: doi:10.1002/anie.200462466
- JUN'ICHI YOSHIDA: "Selective organic synthesis using the micro reactor", Pharmacia, vol. 41, no. 7, 2005, pages 659-663, XP009509092, ISSN: 0014-8601
- JUN'ICHI YOSHIDA: Flow . Micro Gosei -Kiso kara Jissai no Gosei .Seize made, 15 July 2014 (2014-07-15), pages 106-107, XP009510223,

## Description

### TECHNICAL FIELD

The present invention relates to a method of producing an aldehyde compound or a ketone compound.

More specifically, the present invention relates to a method of performing a flow reaction in a flow passage to produce an aldehyde compound or a ketone compound by oxidation of a primary or secondary alcohol compound.

### BACKGROUND ART

Methods of oxidizing a primary or secondary alcohol compound to produce an aldehyde compound or a ketone compound are very important for organic synthesis reactions and also industrially very useful. Oxidizing agents include, for example, chromic acid, manganese dioxide, a hypochlorite, a halogen acid salt, a selenium compound, ruthenium tetroxide (RuO₄), a transition metal catalyst, a pentavalent bismuth compound, silver carbonate, a coppery(II) salt, and lead tetraacetate. Other oxidation reactions include oxidation using dimethyl sulfoxide, oxidation using N-halogenocarboxylic acid amide, Corey-Kim oxidation using dimethyl sulfide and N-chlorosuccinimide as activators, oxidation using a carbonyl compound (Oppenauer oxidation), and oxidation with rearrangement such as pinacol-pinacolone rearrangement.

Examples of oxidation using dimethyl sulfoxide include DMSO-DCC method (Pfitzner-Moffatt method), DNSO-acetic anhydride method (Albright-Goldman method), DMSO-phosphorus pentoxide method (Onodera method), DMSO-sulfur trioxide-pyridine method, and DMSO-oxalyl chloride method and DMSO-trifluoroacetic anhydride method (both corresponding to Swern method).

Among the examples of oxidation using dimethyl sulfoxide, Swern oxidation using dimethyl sulfoxide (DMSO) and trifluoroacetic anhydride as activators is widely used as laboratory-scale oxidation because it does not generate any heavy metal-containing waste, has a wide range of applicable compounds, and does not bring about excessive oxidation.

In Swern oxidation, however, an activated form of the sulfoxide compound (a trifluoroacetoxydimethylsulfonium salt in the case of trifluoroacetic anhydride) and an intermediate such as an alkoxysulfonium salt are thermally unstable. In Swern oxidation, therefore, the reaction temperature should be kept at -50°C or lower. Otherwise, Pummerer rearrangement can occur at -30°C or higher to generate by-products.

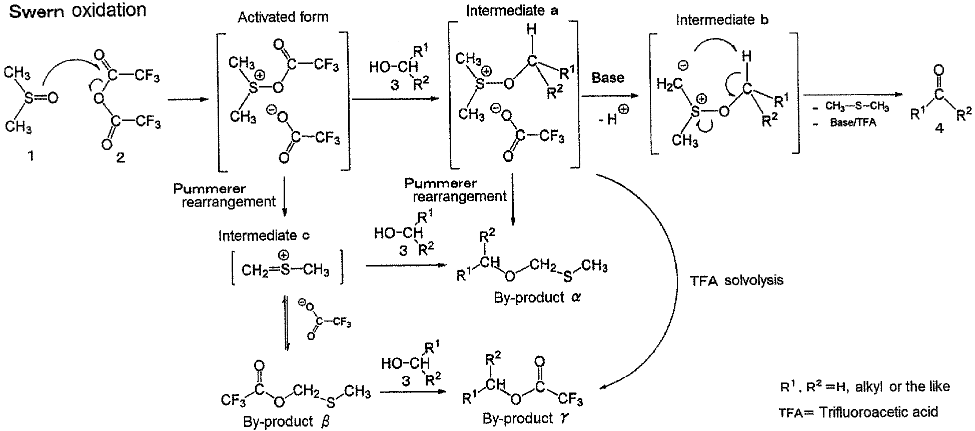

Industrially useful aldehyde compounds or ketone compounds include not only simple compounds as industrial intermediates, which are used in large amounts, but also aldehyde compounds or ketone compounds with a variety of functions, which generally have complicated structures.

In Swern oxidation, the reaction temperature needs to be strictly controlled as mentioned above, and in addition the number of types of by-products will increase, as is also apparent from the reaction scheme above, if the primary or secondary alcohol compound as the substrate has a nucleophilic substituent or a substituent vulnerable to nucleophilic attack in addition to the hydroxy group oxidizable into a carbonyl group.

On the other hand, studies have been conducted on performing Swern oxidation in a microreactor (for example, see Patent Literatures 1 and 2, and Non-Patent Literatures 1 and 2).

### CITATION LIST

### PATENT LITERATURES

Patent Literature 1: Japanese Patent No. 4661597
Patent Literature 2: JP-T-2009-542733 (the term "JP-T" means a published Japanese translation of a PCT patent application)

### NON-PATENT LITERATURES

Non-Patent Literature 1: Angew. Chem. Int. Ed., 2005, 44, 2413-2416
Non-Patent Literature 2: Organic Process Research & Development, 2008, 12, 911-920

### SUMMARY OF INVENTION

### TECHNICAL PROBLEM

Under these circumstances, there has been a need for a method capable of producing, with high yield and high purity, an aldehyde compound or a ketone compound that is useful as an industrial intermediate and has a complicated structure. In particular, a need has been felt for the development of a simple production method that is capable of selectively oxidizing only the target hydroxy group without requiring strict low-temperature control and that inhibits the generation of by-products even when an alcohol compound having, in the molecule, a plurality of asymmetric carbon atoms, and a nucleophilic substituent or a substituent vulnerable to nucleophilic attack is used as a raw material.

Therefore, the present invention contemplates providing a method capable of easily producing an aldehyde compound or a ketone compound from a primary or secondary alcohol compound having a nucleophilic substituent or a substituent vulnerable to nucleophilic attack by highly selective oxidation reaction with high yield, high purity, and easy control of reaction temperature.

### SOLUTION TO PROBLEM

As a result of intensive studies on various oxidation methods and reaction procedures, the inventors have found that the problems can be resolved by the following means.
(1) A method of producing an aldehyde compound or a ketone compound by oxidation of a primary or secondary alcohol compound having, in the molecule, a group selected from an amino group, an azido group, a hydroxy group, and a hydroxy group protected with a protective group, the method comprising the steps of:
   a first step including colliding and mixing an acid anhydride having 4 to 10 carbon atoms and a liquid containing the alcohol compound and a dialkyl sulfoxide having 2 to 8 carbon atoms in a flow-through type reactor to cause a reaction; and
   a second step including colliding and mixing, in a flow-through type reactor, a trialkylamine having 3 to 10 carbon atoms and the mixture liquid obtained in the first step to cause a reaction;
   wherein the reactions in the first step and the second step are sequentially performed, and a mixture linear velocity in the first step is in a range of from 1 m/s to 6 m/s.
(2) The production method described in the above item (1), wherein the aldehyde compound or the ketone compound has a group selected from the amino group, the azido group, the hydroxy group, and the hydroxy group protected with a protective group, which are derived from the molecule of the alcohol compound.
(3) The production method described in the above item (1) or (2), wherein the hydroxyl group protected with a protective group is an acyloxy group, a sulfonyloxy group, a sulfonylamino carbonyloxy group, a silyloxy group, an alkoxy ethyleneoxy group, an alkylthio ethyleneoxy group, or a group represented by Formula (p) described below: 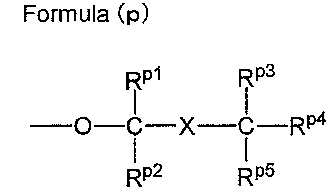 wherein X represents -O- or -S-; R^{p1} to R^{p5} each independently represent a hydrogen atom, an alkyl group, or an aryl group; and R^{p1}, and R^{p2} or R^{p3} may combine together to form a ring.
(4) The production method described in any one of the above items (1) to (3), wherein the alcohol compound has, in its molecule, at least two groups selected from an amino group, an azido group, a hydroxy group, and a hydroxy group protected with a protective group.
(5) The production method described in any one of the above items (1) to (4), wherein the alcohol compound is a cyclic alcohol compound.
(6) The production method described in any one of the above items (1) to (5), wherein the alcohol compound is a cyclic alcohol compound having -C(=O)-, -O-C(=O)- or -C(=O)-O- as ring-constituting atoms or bonds.
(7) The production method described in any one of the above items (1) to (6), wherein the alcohol compound is a cyclic alcohol compound having a 6- or more-membered ring.
(8) The production method described in any one of the above items (1) to (7), wherein the alcohol compound has two or more asymmetric carbon atoms.
(9) The production method described in any one of the above items (1) to (8), wherein the reaction temperature in the first step is from -30 to 30°C.
(10) The production method described in any one of the above items (1) to (9), wherein the reaction temperature in the second step is from -30 to 50°C.

In the present invention, each group specified in each compound or each formula may have a substituent unless otherwise specified, which may be a group selected from the substituent group S.

In the present invention, if a certain compound can have optical isomers, conformational isomers, enantiomers, cis-trans isomers, or other isomers, the compound may be provided in the form of one of the isomeric structures or in the form of any mixture of the isomers.

### ADVANTAGEOUS EFFECTS OF INVENTION

According to the production method of the present invention, an aldehyde compound or a ketone compound is easily produced, by high selective oxidation reaction, from a primary or secondary alcohol compound having a nucleophilic substituent or a substituent vulnerable to nucleophilic attack, with high yield, high purity, and easy control of reaction temperature.

Other and further features and advantages of the invention will appear more fully from the following description, appropriately referring to the accompanying drawing.

### BRIEF DESCRIPTION OF THE DRAWINGS

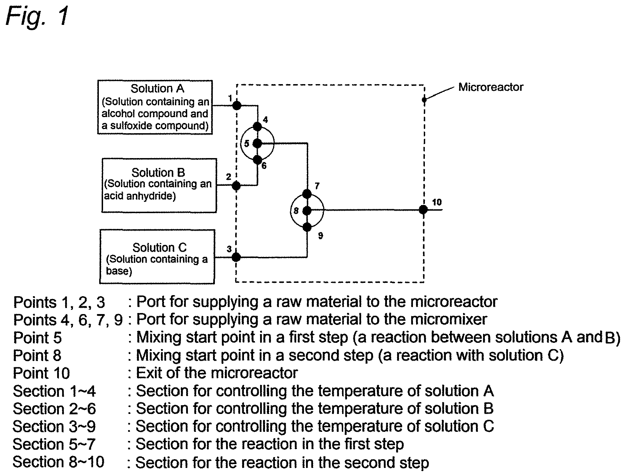

{Fig. 1}
   Fig. 1 is a schematic configuration diagram showing the total configuration of a preferred typical production system according to the present invention.

### MODE FOR CARRYING OUT THE INVENTION

### «Method of producing aldehyde compound or ketone compound»

The present invention is a method of producing an aldehyde compound or a ketone compound by oxidation of a primary or secondary alcohol compound. According to the present invention, an alcohol compound having, in the molecule, a group selected from an amino group, an azido group, a hydroxy group, and a hydroxy group protected with a protective group undergoes selective oxidation of only a specific hydroxy group to a carbonyl group, while by-product generation is suppressed.

Moreover, according to the present invention, reactions are sequentially performed, including: a first step of colliding and mixing an alcohol compound, a dialkyl sulfoxide having 2 to 8 carbon atoms, and an acid anhydride having 4 to 10 carbon atoms in a flow-through type reactor to cause a reaction; and a second step of colliding and mixing, in a microreactor, a trialkylamine having 3 to 10 carbon atoms and the mixture liquid obtained in the first step to cause a reaction.

The present invention is described below in detail.

### <Primary or secondary alcohol compound>

The alcohol compound that is used in the present invention is a primary or secondary alcohol compound having a nucleophilic substituent or a substituent vulnerable to nucleophilic attack.

In the present invention, the nucleophilic substituent or the substituent vulnerable to nucleophilic attack is less likely to be involved in the reaction, and an aldehyde compound or ketone compound can be produced with such a substituent substantially maintained.

### [Nucleophilic substituent and substituent vulnerable to nucleophilic attack]

In the present invention, such a group is selected from an amino group (-NR₂), an azido group (-N₃), a hydroxy group (-OH), and a hydroxy group protected with a protective group.

In this regard, R represents a hydrogen atom or a substituent, and at least one of two R's bound to the nitrogen atom is preferably a substituent.

In the molecule having a simple hydroxy group not protected with any protective group, there is no hydrogen atom on the atom, preferably carbon atom, substituted with the simple hydroxy group.

Preferred examples of the nucleophilic substituent and the substituent vulnerable to nucleophilic attack include an azido group (-N₃), a hydroxy group (-OH), and a hydroxy group protected with a protective group.

The hydroxy group protected with a protective group is preferably an acyloxy group, a sulfonyloxy group, a sulfonylamino carbonyloxy group, a silyloxy group, an alkoxy ethyleneoxy group, an alkylthio ethyleneoxy group, or a group represented by Formula (p) described below.

The acyloxy group may be an aliphatic acyloxy group, an aromatic acyloxy group, or a heterocyclic acyloxy group. The aliphatic acyloxy group, the aromatic acyloxy group, and the heterocyclic acyloxy group may have a substituent.

Examples of the aliphatic acyloxy group include a formyloxy group, an alkylcarbonyloxy group, an alkenylcarbonyloxy group, a cycloalkylcarbonyloxy group, and a cycloalkenylcarbonyloxy group. The number of carbon atoms of the aliphatic acyloxy group is preferably 20 or less, more preferably 12 or less. The lower limit of the number of carbon atoms is 1 or more. In the case of the alkylcarbonyloxy group, the lower limit is 2, and in the case of the alkenylcarbonyloxy group, the lower limit is 3. In the case of the cycloalkylcarbonyloxy group, the lower limit of the number of carbon atoms is 4, preferably 6 to 8, more preferably 7. In the case of the cycloalkenylcarbonyloxy group, the lower limit is 6, preferably 6 to 7, more preferably 7.

Examples of the aliphatic acyloxy group include acetyloxy, propionyloxy, pivaloyloxy, lauroyloxy, stearoyloxy, acryloyloxy, methacryloyloxy, crotonoyloxy, oleyloxy, cinnamoyloxy, cyclopropane carbonyloxy, cyclopentane carbonyloxy, cyclohexane carbonyloxy, cyclopentene carbonyloxy, and cyclohexene carbonyloxy.

The aromatic acyloxy group is an aromatic ring carbonyloxy group composed of a hydrocarbon ring, and may have a substituent. The number of carbon atoms is preferably 7 to 21, more preferably 7 to 13.

Examples of the aromatic acyloxy group include benzoyloxy, toluoyloxy, p-toluoyloxy, and naphthoyloxy.

The heterocycle of the heterocyclic acyloxy group may have a substituent, and may be any one of a heterocyclic aromatic ring, a non-aromatic unsaturated heterocycle, and a saturated heterocycle. The hetero atom constituting the heterocycle is preferably an atom selected from an oxygen atom, a nitrogen atom and a sulfur atom. Further, the heterocycle is preferably a 5- or 6-membered heterocycle, and may be condensed with a benzene ring or a heterocycle.

The number of carbon atoms of the heterocyclic acyloxy group is preferably 1 to 20, more preferably 2 to 16, and further preferably 3 to 12.

Examples of the heterocyclic acyloxy group include furoyloxy, tenoyloxy, nicotinoyloxy, isonicotinoyloxy, and piperidine carbonyloxy.

The sulfonyloxy group may be an aliphatic sulfonyloxy group, an aromatic sulfonyloxy group, or a heterocyclic sulfonyloxy group. The aliphatic sulfonyloxy group, the aromatic sulfonyloxy group, and the heterocyclic sulfonyloxy group may have a substituent.

Examples of the aliphatic sulfonyloxy group include an alkylsulfonyloxy group, an alkenylsulfonyloxy group, a cycloalkylsulfonyloxy group, and a cycloalkenylsulfonyloxy group. The number of carbon atoms of the aliphatic sulfonyloxy group is preferably 20 or less, more preferably 12 or less. The lower limit of the number of carbon atoms is 1 or more. In the case of the alkylsulfonyloxy group, the lower limit is 1, and in the case of the alkenylsulfonyloxy group, the lower limit is 2. In the case of the cycloalkylsulfonyloxy group, the lower limit of the number of carbon atoms is 3, preferably 4 to 6, more preferably 5. In the case of the cycloalkenylsulfonyloxy group, the lower limit is 5, preferably 5 to 6.

Examples of the aliphatic acyloxy group include methane sulfonyloxy, ethane sulfonyloxy, propane sulfonyloxy, octane sulfonyloxy, 2-ethylhexane sulfonyloxy, hexadecane sulfonyloxy, propene sulfonyloxy, cyclopentane sulfonyloxy, cyclohexane sulfonyloxy, cyclopentene sulfonyloxy, and cyclohexene sulfonyloxy.

The aromatic sulfonyloxy group is an aromatic ring sulfonyloxy group composed of a hydrocarbon ring, and may have a substituent. The number of carbon atoms is preferably 6 to 20, more preferably 6 to 12.

Examples of the aromatic sulfonyloxy group include benzene sulfonyloxy, toluene sulfonyloxy, and naphthalene sulfonyloxy.

Examples of the heterocycle of the heterocyclic sulfonyloxy group include those exemplified as the heterocycle of the heterocyclic acyloxy group, and a preferable range is also the same.

The number of carbon atoms of the heterocyclic sulfonyloxy group is preferably 0 to 20, more preferably 1 to 16, and further preferably 2 to 12.

Examples of the heterocyclic sulfonyloxy group include pyridine sulfonyloxy, and piperidine sulfonyloxy.

The sulfonylamino carbonyloxy group may be an aliphatic sulfonylamino carbonyloxy group, an aromatic sulfonylamino carbonyloxy group, or a heterocyclic sulfonylamino carbonyloxy group. The aliphatic sulfonylamino carbonyloxy group, the aromatic sulfonylamino carbonyloxy group, and the heterocyclic sulfonylamino carbonyloxy group may have a substituent. The preferred range of the sulfonyl moiety of the sulfonylaminocarbonyloxy group is the same as that of the sulfonyl moiety of the sulfonyloxy group.

Examples of the sulfonylamino carbonyloxy group include methanesulfonylamino carbonyloxy, benzenesulfonylamino carbonyloxy, p-toluenesulfonylamino carbonyloxy, and naphthalenesulfonylamino carbonyloxy.

The silyloxy group is preferably a silyloxy group having a substituent on the silicon atom. Examples of such a substituent include an alkyl group, an alkenyl group, a cycloalkyl group, a cycloalkenyl group, an aryl group, a heterocyclic group, an alkoxy group, an alkenyloxy group, a cycloalkoxy group, a cycloalkenyloxy group, an aryloxy group, and a heterocyclic oxy group. The number of carbon atoms is preferably 3 to 20.

Examples of the silyloxy group include trimethylsilyloxy, triethylsilyloxy, benzyldimethylsilyloxy, t-butyldimethylsilyloxy, phenyldimethylsilyloxy, triphenylsilyloxy, trimethoxysilyloxy, and triphenoxysilyloxy.

The number of carbon atoms of the alkoxy ethyleneoxy group is preferably 3 to 20, and more preferably 3 to 12.

Examples of the alkoxy ethyleneoxy group include methoxy ethyleneoxy, ethoxy ethyleneoxy, isopropoxy ethyleneoxy, t-butoxy ethyleneoxy, and cyclohexyloxy ethyleneoxy.

The number of carbon atoms of the alkylthio ethyleneoxy group is preferably 3 to 20, and more preferably 3 to 12.

Examples of the alkylthio ethyleneoxy group include methylthio ethyleneoxy, ethylthio ethyleneoxy, isopropylthio ethyleneoxy, t-butylthio ethyleneoxy, and cyclohexylthio ethyleneoxy.

Besides the above, the hydroxy group protected with a protective group is preferably a group represented by Formula (p) below.

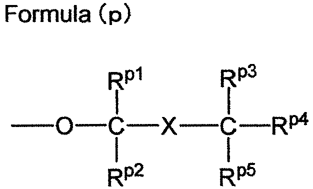

In Formula (p), X represents -O- or -S-, and R^{p1} to R^{p5} each independently represent a hydrogen atom, an alkyl group, or an aryl group. Herein, R^{p1}, and R^{p2} or R^{p3} may combine together to form a ring.

The number of carbon atoms of the alkyl group of R^{p1} to R^{p5} is preferably 1 to 20, and more preferably 1 to 12. Examples of the alkyl group include methyl, ethyl, isopropyl, butyl, t-butyl, hexyl, octyl, 2-ethylhexyl, decyl, dodecyl, hexadecyl, and octadecyl. The alkyl group may have a substituent.

The number of carbon atoms of the aryl group of R^{p1} to R^{p5} is preferably 6 to 20, and more preferably 6 to 12. Examples of the aryl group include phenyl and naphthyl. The aryl group may have a substituent.

The ring formed by combining of R^{p1} with R^{p2} or R^{p3} is preferably a 5- or 6-membered ring, and may have a substituent. Preferred examples of such a substituent include an alkyl group, an aryl group, a heterocyclic group, an alkoxy group, a heterocyclic oxy group, an alkylamino group, an arylamino group, a heterocyclic amino group, an acylamino group, a hydroxy group, an acyloxy group, a sulfonyloxy group, a silyloxy group, an acyl group, and an onio group (such as ammonio, trialkylammonio and the like).

R^{p1} and R^{p2} may combine together to form a ring, which may be a hydrocarbon ring or a heterocycle and is preferably a heterocycle containing an oxygen atom as a ring constituting atom.

Examples of the ring formed by combining of R^{p1} with R^{p2} include a cyclopentane ring, a cyclohexane ring, a tetrahydrofuran ring, a tetrahydropyran ring, a tetrahydro-4H-pyran-4-one ring, a tetrahydrothiophene ring, a pentamethylene sulfide ring, and a 4-oxothiane ring.

Examples of the ring formed by combining of R^{p1} with R^{p3} include a tetrahydrofuran ring, a tetrahydropyran ring, a tetrahydro-4H-pyran-4-one ring, a tetrahydrothiophene ring, a pentamethylene sulfide ring, and a 4-oxothiane ring.

In the present invention, the hydroxyl group protected with a protective group is preferably an acyloxy group, a sulfonyloxy group, a silyloxy group, an alkoxy ethyleneoxy group, an alkylthio ethyleneoxy group, or a group represented by Formula (p); and more preferably an acyloxy group, or a group represented by Formula (p).

In the present invention, the alcohol compound preferably has, in the molecule, two or more, more preferably three or more groups selected from an amino group, an azido group, a hydroxy group, and a hydroxy group protected with a protective group.

Among these substituents, an azido group and a hydroxy group protected with a protective group are further preferred.

### [Basic structure of primary or secondary alcohol compound]

The primary or secondary alcohol compound for use in the present invention may have any basic structure as long as it has the above-described nucleophilic substituent or substituent vulnerable to nucleophilic attack.

For example, the primary or secondary alcohol compound may be a chain alcohol compound or a cyclic alcohol compound.

The primary or secondary alcohol compound for use in the present invention is preferably a compound represented by Formula (A) below, from which an aldehyde compound or a ketone compound represented by Formula (B) below can be produced by oxidation reaction.

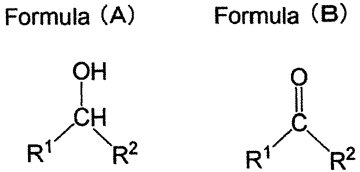

In Formulas (A) and (B), R¹ represents a hydrogen atom, an alkyl group, a cycloalkyl group, an alkenyl group, a cycloalkenyl group, an aryl group, or a heterocyclic group. R² represents an alkyl group, a cycloalkyl group, an alkenyl group, a cycloalkenyl group, an aryl group, or a heterocyclic group. Herein, R¹ and R² may combine together to form a ring. Moreover, each group of R¹ and R² may have a substituent.

The alkyl group of R¹ and R² is a linear or branched alkyl group having preferably 1 to 18 carbon atoms, more preferably 1 to 12 carbon atoms.

Examples of the alkyl group include methyl, ethyl, propyl, isopropyl, butyl, pentyl, hexyl, heptyl, octyl, 2-ethylhexyl, nonyl, decyl, undecyl, dodecyl, tridecyl, tetradecyl, pentadecyl, hexadecyl, heptadecyl, and octadecyl.

The cycloalkyl group of R¹ and R² is preferably a 3- to 18-membered cycloalkyl group, more preferably a 3- to 10-membered cycloalkyl group, further preferably a 3- to 7-membered cycloalkyl group, and particularly preferably a 5- or 6-membered cycloalkyl group. The number of carbon atoms of the cycloalkyl group is preferably 3 to 18, more preferably 5 to 12.

Examples of the cycloalkyl group include cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentadecyl, cyclohexadecyl, cycloheptadecyl, and cyclooctadecyl.

The alkenyl group of R¹ and R² is a linear or branched alkenyl group having preferably 2 to 18 carbon atoms, more preferably 2 to 12 carbon atoms.

Examples of the alkenyl group include vinyl, allyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, hexadienyl, dodecadienyl, octatrienyl, and tetradecatrienyl.

The cycloalkenyl group of R¹ and R² is preferably a 5- to 18-membered cycloalkenyl group, more preferably a 5- to 10-membered cycloalkenyl group, further preferably a 5- to 7-membered cycloalkenyl group, and particularly preferably a 5- or 6-membered cycloalkenyl group. The number of carbon atoms of the cycloalkenyl group is preferably 5 to 18, more preferably 5 to 12.

Examples of the cycloalkenyl group include cyclopentenyl, cyclohexenyl, cycloheptynyl, and cyclooctynyl.

The number of carbon atoms of the aryl group of R¹ and R² is preferably 6 to 14, more preferably 6 to 10. Examples thereof include a monocyclic or bicyclic aryl group, such as phenyl, tolyl, xylyl, naphthyl and phenanthryl.

The heterocyclic group of R¹ and R² is preferably a 5- to 18-membered heterocyclic group, more preferably a 5- to 10-membered heterocyclic group. The hetero atom constituting the heterocycle is preferably an atom selected from a nitrogen atom, an oxygen atom and a sulfur atom. The number of hetero atoms constituting the heterocycle may be 2 or more. The heterocycle of the heterocyclic group may be condensed with an aryl ring, such as a benzene ring, and a naphthalene ring; an aliphatic hydrocarbon ring, such as a cyclopentane ring, a cyclohexane ring, a cyclopentene ring, and a cyclohexene ring; a heterocycle, or the like.

Examples of the heterocyclic group include imidazolyl, oxazolyl, triazolyl, thiazolyl, 1,3,4-thiadiazolyl, pyridyl, pyrimidyl, pyrazyl, furyl, pyranyl, chromanyl, tetrahydropyranyl, thienyl, benzothiazolyl, benzoxazolyl, quinolyl, and acridinyl. Other examples thereof include 5- to 16-membered lactone rings, such as 2-oxo-oxetanyl, 2-oxo-oxolanyl, 2-oxo-chromenyl, 2-oxo-isochromenyl, 2-oxo-oxepanyl, 2-oxo-oxacyclodecanyl, 2-oxo-oxacycloundecanyl, 2-oxo-oxacyclododecanyl, 2-oxo-oxacyclotridecanyl, 2-oxo-oxacyclotetradecanyl, 2-oxo-oxacyclopentadecanyl, and 2-oxo-oxacyclohexadecanyl.

Examples of the ring formed by combining of R¹ and R² in combination include a saturated or unsaturated aliphatic carbon ring, and a saturated or unsaturated heterocycle. The formed ring is preferably a 3- to 18-membered ring. In the case of the heterocycle, the hetero atom constituting the heterocycle is preferably an atom selected from a nitrogen atom, an oxygen atom and a sulfur atom, and the number of hetero atoms constituting the heterocycle may be 2 or more.

Among these, the heterocycle is preferably a 5- to 18-membered ring. In the case of the lactone ring, a 4- to 18-membered ring is preferable.

Examples of the ring formed by combining of R¹ and R² in combination include the following rings.

### 1) 3- to 18-membered saturated ring

### - Monocycle

Examples thereof include a cyclopropane ring, a cyclobutane ring, a cyclopentane ring, a cyclohexane ring, a cycloheptane ring, a cyclooctane ring, a cyclononane ring, a cyclodecane ring, a cycloundecane ring, a cyclododecane ring, a cyclotridecane ring, a cyclotetradecane ring, a cyclopentadecane ring, cyclohexadecane, a cycloheptadecane ring, and a cyclooctadecane ring.

### - Condensed polycycle

Examples thereof include a 1,2,3,4-tetrahydronaphthalene ring, a 9,10-dihydroanthracene ring, and a tetrahydroanthracene ring.

### 2) 5- to 18-membered partially saturated ring

Examples thereof include a cyclopropene ring, a cyclobutene ring, a cyclopentene ring, a cyclohexene ring, a cycloheptene ring, a cyclooctadiene ring, a cyclononene ring, a cyclodecene ring, a cycloundecene ring, a cyclododecatriene ring, a cyclotridecene ring, a cyclotetradecadiene ring, a cyclopentadecene ring, a cyclohexadecene ring, a cycloheptadecene ring, and a cyclooctadecene ring.

### 3) 4- to 18-membered heterocycle

Examples thereof include an oxolane ring; a thiopyran ring; a crown ether ring, such as a 12-crown-4-ether ring, and a 15-crown-5-ether ring; and a lactone ring, such as a β-propiolactone ring, a γ-butyroIactone ring, and a δ-valerolactone ring.

In the present invention, a cyclic alcohol compound having -C(=O)-, -O-C(=O)-, or -C(=O)-O- as ring-constituting atoms or bonds is preferred, in which the ring is preferably a 6- or more-membered ring.

In addition, such a cyclic alcohol compound preferably has a hydroxy group which is oxidizable to a carbonyl group, and the group selected from an amino group, an azido group, a hydroxy group, and a hydroxy group protected with a protective group, that is attached to the basic ring skeleton represented by any one of the ring structures A to I below.

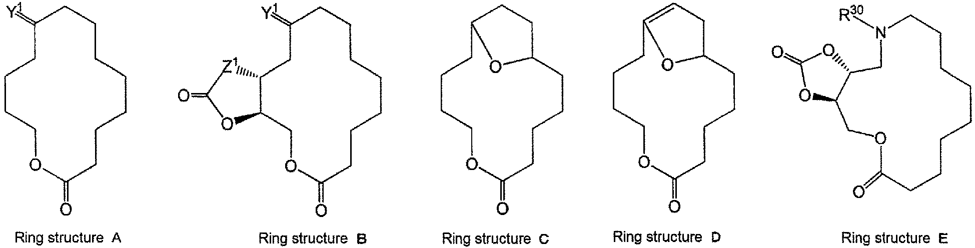

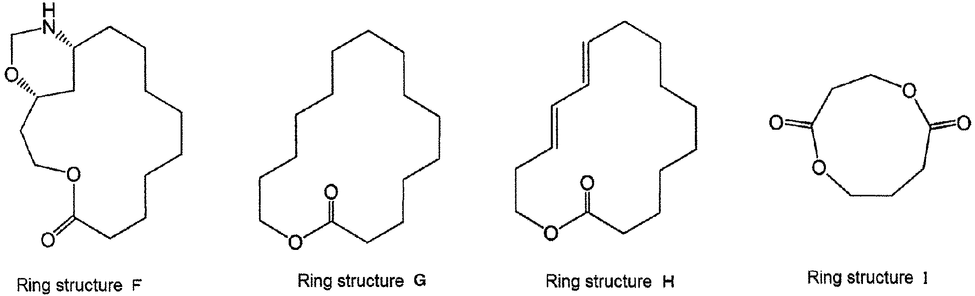

Herein, in the ring structures A and B, Y¹ represents =O or =N-R¹⁰. In the ring structure B, Z¹ represents -O- or -N(R²⁰)-. R¹⁰, R²⁰ and R³⁰ each independently represent a hydrogen atom or a substituent.

Any of the above ring structures may contain an unsaturated bond such as a double bond in the ring structure, may be fused with another ring (preferably a heterocycle), or may have a divalent group such as oxo or imino as a substituent. Ring structures containing these moieties are separately shown above so that particularly preferable structures can be shown clearly.

As the substituent of R¹⁰, an alkyl group and an aryl group are preferable, and an alkyl group is more preferable.

As the substituent of R²⁰, an alkyl group, an aryl group, an alkoxy group, and an aryloxy group are preferable; an alkyl group and an alkoxy group are more preferable; and an alkoxy group is further preferable.

As the substituent of R³⁰, an alkyl group, an aryl group, an acyloxy group, and an alkoxy group are preferable; and an alkyl group and an acyloxy group are more preferable.

In this regard, R¹⁰, R²⁰, and R³⁰ may have a further substituent not capable of being involved in the reaction.

Moreover, in the present invention, an alcohol compound having an asymmetric carbon atom is preferable. The number of asymmetric carbon atoms is preferably 1 or more, more preferably 2 or more, further preferably 3 or more, and particularly preferably 4 or more. The upper limit of the number of asymmetric carbon atoms is not particularly limited, but it is preferably 50 or less, more preferably 30 or less, and further preferably 20 or less.

The alcohol compound with an asymmetric carbon atom can have optical isomers. The alcohol compound may be one of such isomers or a mixture of such isomers. In the present invention, stereocontrol makes it possible to produce the aldehyde compound or the ketone compound in the form of a specific optical isomer from the alcohol compound in the form of a specific optical isomer.

Specific examples of the primary or secondary alcohol compound used in the present invention are described below. However, the present invention is not limited to these examples.

In the following examples, "Ac" represents an acetyl group [-C(=O)-CH₃], "Bz" represents a benzoyl group [-C(=O)-C₆H₅], and "Ts" represents a tosyl group [-SO₂C₆H₅-CH₃-p].

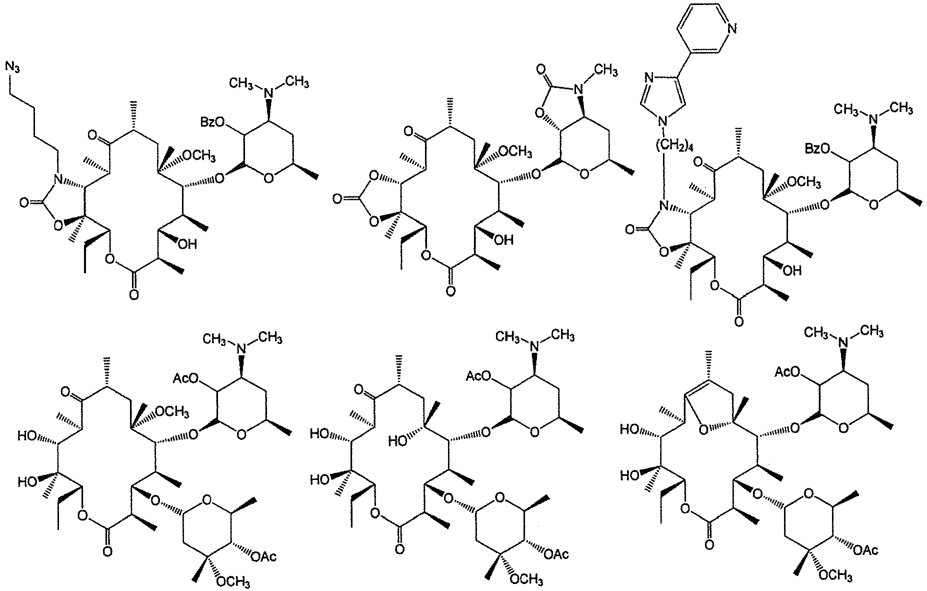

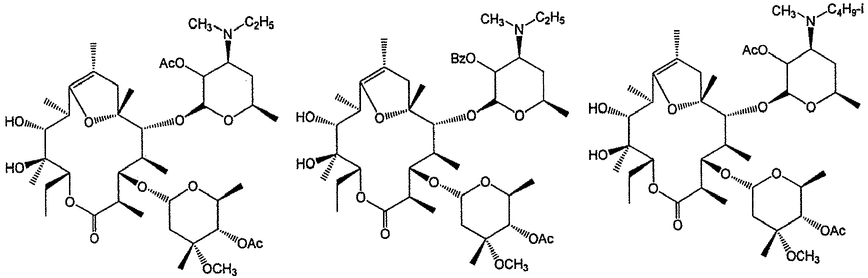

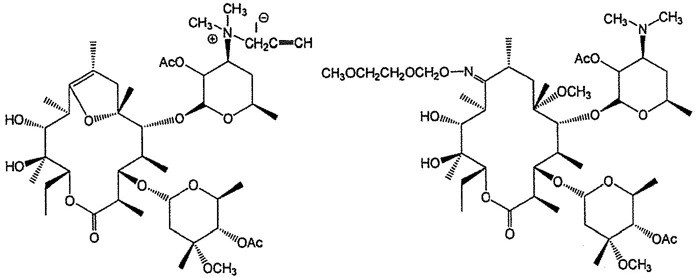

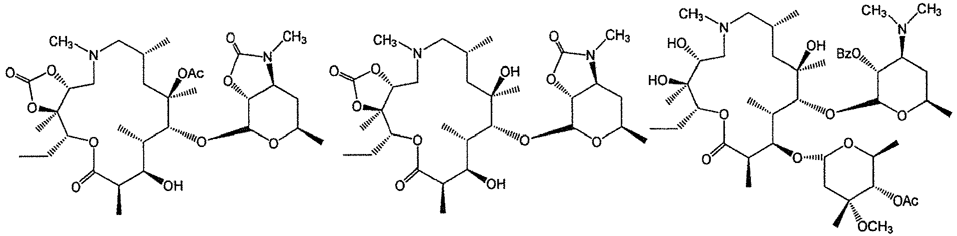

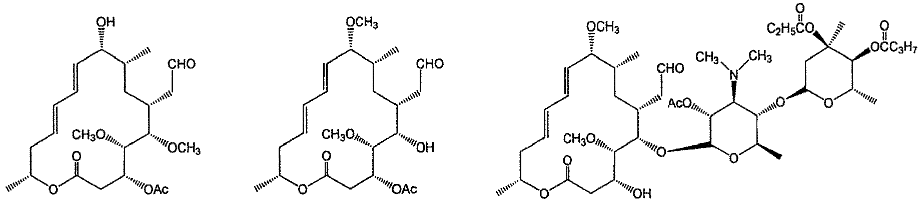

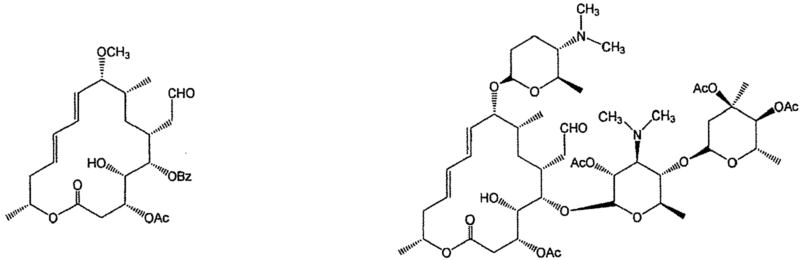

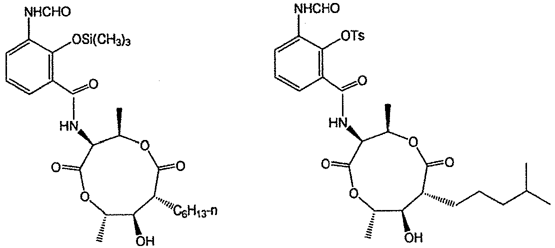

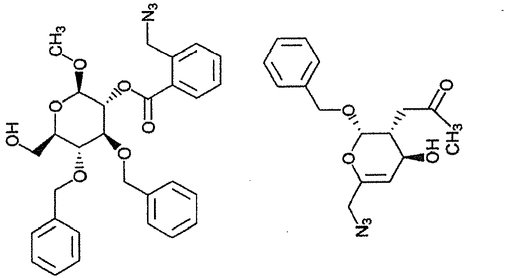

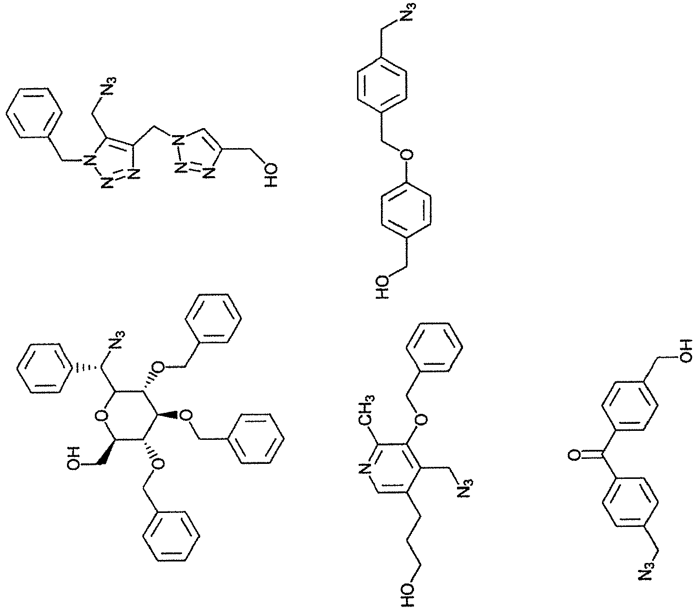

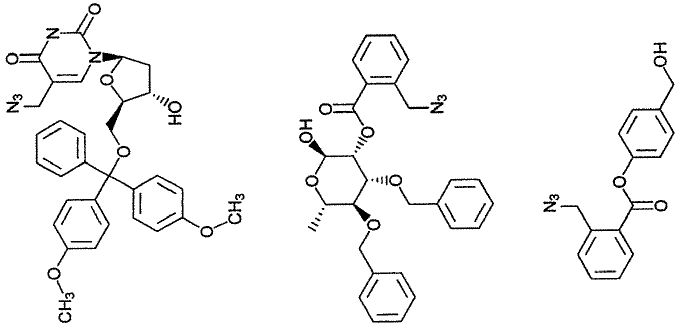

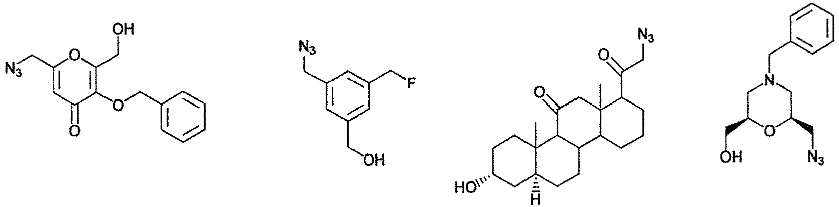

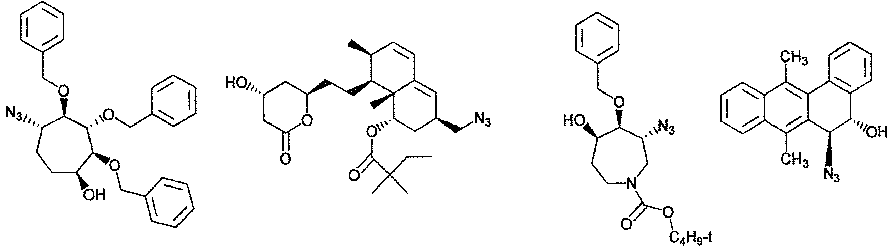

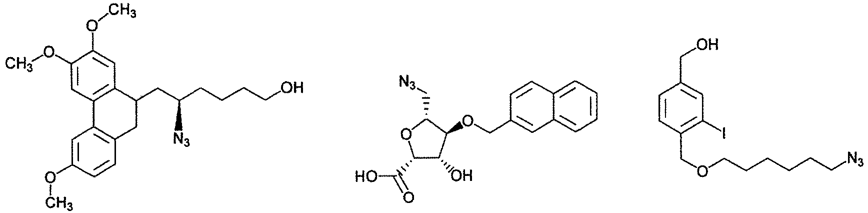

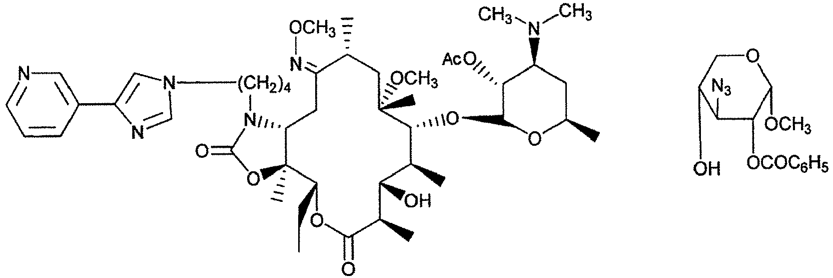

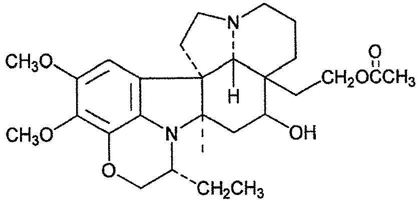

The primary or secondary alcohol compound having a group selected from an amino group, an azido group, a hydroxyl group, and a hydroxyl group protected with a protective group, which is used in the present invention, can be synthesized by a known method, for example, a method described in Journal of Antibiotics, 1996, 49, 493-495; Tetrahedron Letters, 2005, 46, 1483-1487; European Journal of Medicinal Chemistry, 2013, 69, 174-181; or the like.

### <Aldehyde compound and ketone compound>

In the present invention, the aldehyde compound or the ketone compound is produced from the primary or secondary alcohol compound having, in the molecule, a group selected from an amino group, an azido group, a hydroxy group, and a hydroxy group protected with a protective group. In the production method of the present invention, the group of the primary or secondary alcohol compound selected from an amino group, an azido group, a hydroxy group (with no hydrogen atom on the carbon atom substituted with the hydroxy group), and a hydroxy group protected with a protective group, other than the hydroxy group oxidizable to a carbonyl group, can be kept unchanged before and after the reaction.

The other moieties of the aldehyde compound or the ketone compound produced in the present invention than the carbonyl group (-CH(=O) or -C(=O)-) formed by oxidation of the reactive hydroxy group of the primary or secondary alcohol compound raw material have the same preferred range of the primary or secondary alcohol compound. Therefore, the same applies here as described above for the aldehyde compound or ketone compound represented by Formula (B).

Examples of the aldehyde compound or the ketone compound produced in the present invention include those derived from the above examples of the primary or secondary alcohol compound raw material by replacing >CH-OH with >C(=O). Therefore, except for the oxidizable hydroxy group, the configuration of the asymmetric carbon atom will remain, and stereocontrol will result in the same optical isomer.

For example, in the case of the following alcohol compound, a specific example of the ketone compound is a compound described below.

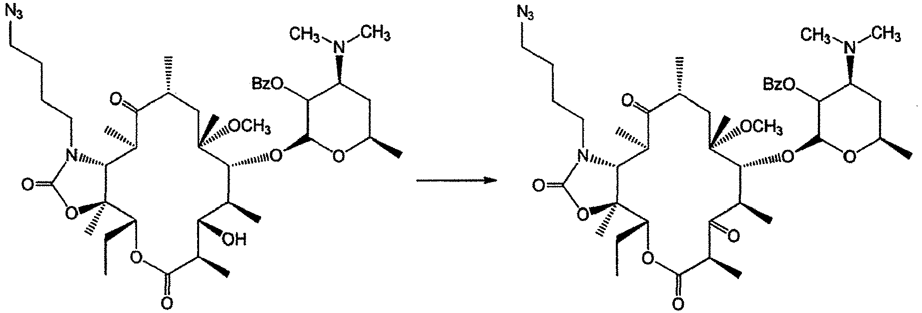

### <Reaction reagents>

The primary or secondary alcohol compound is oxidized in the production method of the present invention. The oxidation is performed by Swern oxidation or a method similar thereto. Therefore, a dialkyl sulfoxide, an acid anhydride, and a base are used as reaction reagents.

### (Dialkyl sulfoxide)

In the present invention, a dialkyl sulfoxide having 2 to 8 carbon atoms is used.

The sulfoxide compound is preferably represented by Formula (SO) described below.

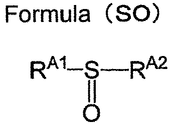

In Formula (SO), R^{A1} and R^{A2} each independently represent an alkyl group.

Herein, the total number of carbon atoms of R^{A1} and R^{A2} is 2 to 8.

The alkyl group of R^{A1} and R^{A2} is a linear or branched alkyl group, and may have a substituent.

Examples of the alkyl group include methyl, ethyl, propyl, isopropyl, butyl, isobutyl, t-butyl, pentyl, hexyl, and heptyl.

Examples of the dialkyl sulfoxide compound having 2 to 8 carbon atoms include dimethyl sulfoxide, diethyl sulfoxide, dipropyl sulfoxide, diisopropyl sulfoxide, dibutyl sulfoxide, ethylmethyl sulfoxide, propylmethyl sulfoxide, isopropylmethyl sulfoxide, butylmethyl sulfoxide, butylethyl sulfoxide, butylpropyl sulfoxide, and butylisopropyl sulfoxide. Among these, dimethyl sulfoxide, diethyl sulfoxide, dipropyl sulfoxide, and dibutyl sulfoxide are preferable; and dimethyl sulfoxide is more preferable.

Various sulfoxide compounds are commercially available and can be obtained. Alternatively, the sulfoxide compound can be synthesized in accordance with a known method (e.g., a method described in Tetrahedron Lett., vol. 43, p. 5177-5179 (2002), or the like).

The amount of the sulfoxide compound to be used is preferably 1.0 to 5.0 molar equivalents, more preferably 1.5 to 4.5 molar equivalents, further more preferably 2.0 to 4.0 molar equivalents, based on the amount of the raw material alcohol compound.

### (Acid anhydride)

In the present invention, an acid anhydride having 4 to 10 carbon atoms is used.

The acid anhydride may be any of an aliphatic carboxylic acid anhydride, an aromatic carboxylic acid anhydride, a heterocyclic carboxylic acid anhydride, and any mixture of these carboxylic acid anhydrides.

The acid anhydride is preferably an acid anhydride of an aliphatic carboxylic acid, more preferably an acid anhydride of an aliphatic carboxylic acid substituted with a halogen atom, and further preferably an acid anhydride of an aliphatic carboxylic acid substituted with a fluorine atom.

Examples of the acid anhydride include acetic anhydride, butyric anhydride, isobutyric anhydride, pivalic anhydride, trifluoroacetic anhydride, heptafluoroacetic anhydride, and trichloroacetic anhydride. Among these, acetic anhydride, trifluoroacetic anhydride, and trichloroacetic anhydride are preferable; trifluoroacetic anhydride and trichloroacetic anhydride are more preferable.

The amount of the acid anhydride to be used is preferably 1.0 to 3.0 molar equivalents, more preferably 1.1 to 2.8 molar equivalents, further more preferably 1.2 to 2.5 molar equivalents, based on the amount of the raw material alcohol compound.

### (Base)

In the present invention, a trialkylamine having 3 to 10 carbon atoms is used as a base.

The three alkyl substituents on the nitrogen atom may be the same or different. Each alkyl group may be a linear or branched alkyl group and have a substituent. Examples thereof include methyl, ethyl, propyl, isopropyl, butyl, isobutyl, t-butyl, pentyl, hexyl, heptyl, octyl, and 2-ethylhexyl.

Examples of the trialkylamine having 3 to 10 carbon atoms include trimethyl amine, dimethyl ethyl amine, diethyl methyl amine, triethyl amine, diethyl methyl amine, dimethyl isopropyl amine, dipropyl ethyl amine, diisopropyl ethyl amine, butyl dimethyl amine, and butyl diisopropyl amine.

Among these, triethyl amine and diisopropyl ethyl amine are preferable.

The amount of the base to be used is preferably 1.2 to 6.0 molar equivalents, more preferably 1.3 to 5.5 molar equivalents, further more preferably 1.5 to 5.0 molar equivalents, based on the amount of the raw material alcohol compound.

The base is mixed after the raw material alcohol compound, the dialkyl sulfoxide, and the acid anhydride are mixed together.

### (Reaction solvent)

The reaction solvent may be any type capable of dissolving the substrate and not undergoing any side reaction.

A single solvent or a mixture of two or more solvents may be used.

Examples of the reaction solvent include a ketone-series solvent, such as acetone, ethyl methyl ketone, methyl isopropyl ketone, diisopropyl ketone, methyl isobutyl ketone, and diisobutyl ketone; an ether-series solvent, such as diethyl ether, diisopropyl ether, methyl-tert-butyl ether, tetrahydrofuran (THF), and dioxane; an amide-series solvent, such as dimethylformamide, dimethylacetamide, N-methyl-2-pyrolidone, tetramethylurea, and 1,3-dimethyl imidazolinone; a sulfur-containing solvent, such as dimethyl sulfoxide and sulfolane; a nitrile-series solvent, such as acetonitrile; a glycol-series solvent, such as ethylene glycol dimethyl ether, propylene glycol diethyl ether, diethylene glycol dimethyl ether, diethylene glycol ethyl methyl ether, ethylene glycol diacetate, ethylene glycol distearate, ethylene glycol diacrylate, and diethylene glycol diacetate; a halogen-series solvent, such as methylene chloride, chloroform, and chlorobenzene; an ester-series solvent, such as methyl acetate, ethyl acetate, isopropyl acetate, and butyl acetate; an aromatic hydrocarbon-series solvent, such as toluene, xylene, mesitylene, and tetrahydronaphthalene; and a terpene-series solvent, such as terpinene, terpinolene, cymene, and phellandrene.

Among these, acetone, THF, acetonitrile, methylene chloride, ethyl acetate, and toluene are preferable.

The amount of the solvent to be used is generally 1 to 300 mL, preferably 1 to 150 mL, more preferably 5 to 100 mL, based on 1 g of the raw material alcohol compound, through it depends on the substrate and the reaction conditions.

In the present invention, unless otherwise specified, each substituent, which each group in each Formula or each compound is further capable of having, is preferably selected from the substituent group S shown below.

### [Substituent group S]

Examples thereof include: an alkyl group (preferably an alkyl group having 1 to 20 carbon atoms, e.g. methyl, ethyl, isopropyl, t-butyl, pentyl, heptyl, 1-ethylpentyl, 2-ethylhexyl, benzyl, 2-ethoxyethyl, or 1-carboxymethyl), an alkenyl group (preferably an alkenyl group having 2 to 20 carbon atoms, e.g. vinyl, allyl, or oleyl), an alkynyl group (preferably an alkynyl group having 2 to 20 carbon atoms, e.g. ethynyl, 2-butynyl, or phenylethynyl), a cycloalkyl group (preferably a cycloalkyl group having 3 to 20 carbon atoms, e.g. cyclopropyl, cyclopentyl, cyclohexyl, or 4-methylcyclohexyl), an aryl group (preferably an aryl group having 6 to 26 carbon atoms, e.g. phenyl, 1-naphthyl, 2-naphthyl, 4-methoxyphenyl, 2-chlorophenyl, or 3-methylphenyl), a heterocyclic group (preferably a heterocyclic group having 0 to 20 carbon atoms, of which the hetero atom constituting the ring is selected from an oxygen atom, a nitrogen atom, and a sulfur atom; the group may be a 5- or 6-membered ring and condensed with a benzene ring or a heterocycle; and the heterocycle of the heterocyclic group may be a saturated ring, an unsaturated ring or an aromatic ring; and heterocycle group may be, e.g. 2-pyridyl, 4-pyridyl, 2-imidazolyl, 2-benzimidazolyl, 2-thiazolyl, or 2-oxazolyl), an alkoxy group (preferably an alkoxy group having 1 to 20 carbon atoms, e.g. methoxy, ethoxy, isopropyloxy, or benzyloxy), an aryloxy group (preferably an aryloxy group having 6 to 26 carbon atoms, e.g. phenoxy,1-naphthyloxy, 2-naphthyloxy, 3-methylphenoxy, or 4-methoxyphenoxy), a heterocyclic oxy group (preferably a heterocyclic oxy group of the heterocyclic group described above),
an alkylthio group (preferably an alkylthio group having 1 to 20 carbon atoms, e.g. methylthio, ethylthio, isopropylthio, or benzylthio), an arylthio group (preferably an arylthio group having 6 to 26 carbon atoms, e.g. phenylthio, 1-naphthylthio, 2-naphthylthio, 3-methylphenylthio, or 4-methoxyphenylthio), an acyl group (preferably an acyl group having 20 or less carbon atoms including a formyl group, an alkylcarbonyl group, an alkenylcarbonyl group, an arylcarbonyl group, and a heterocyclic carbonyl group, e.g. acetyl, pivaloyl, acryloyl, methacryloyl, benzoyl or nicotinoyl), an alkoxycarbonyl group (preferably an alkoxycarbonyl group having 2 to 20 carbon atoms, e.g. ethoxycarbonyl, or 2-ethylhexyloxycarbonyl), an aryloxycarbonyl group (preferably an aryloxycarbonyl group having 7 to 20 carbon atoms, e.g. phenyloxycarbonyl, or naphthyloxycarbonyl), an amino group (preferably an amino group having 0 to 20 carbon atoms including an amino group, an alkylamino group, an arylamino group, and a heterocyclic amino group, e.g. amino, N,N-dimethylamino, N,N-diethylamino, N,N-diphenylamino, N-ethylamino, anilino, 1-pyrrolidine-1-yl, piperidine-1-yl, morpholine-1-yl, or thiomorpholine-1-yl), a sulfamoyl group (preferably a sulfamoyl group having 0 to 20 carbon atoms including an alkylsulfamoyl group and an arylsulfamoyl group, e.g. sulfamoyl, N,N-dimethylsulfamoyl, or N-phenylsulfamoyl), an acyloxy group (preferably an acyloxy group having 1 to 20 carbon atoms, e.g. acetyloxy, or benzoyloxy), a carbamoyl group (preferably a carbamoyl group having 1 to 20 carbon atoms including an alkylcarbamoyl group and an arylcarbamoyl group, e.g. carbamoyl, N,N-dimethylcarbamoyl, or N-phenylcarbamoyl), an acylamino group (preferably an acylamino group having 1 to 20 carbon atoms, e.g. acetylamino, acryloylamino, benzoylamino, or nicotinamide), a cyano group, a nitro group, a hydroxy group, a mercapto group, a carboxy group, a sulfo group, a phosphate group, a halogen atom (e.g. fluorine atom, chlorine atom, bromine atom, or iodine atom), a silyloxy group (preferably the silyloxy group in the hydroxyl group protected with a protective group described above), and an azido group (-N₃). Other examples thereof include an oxo group (=O) of a divalent group, a divalent imino group (a group having =N-, including an alkylimino group and an alkoxyimino group), a sulfonyloxy group (preferably the sulfonyloxy group in the hydroxyl group protected with a protective group described above), and a sulfonylamino carbonyloxy group (preferably the sulfonylamino carbonyloxy group in the hydroxyl group protected with a protective group described above).

These substituents may be further substituted with the substituent described above.

### <Reaction steps>

In the present invention, reactions are sequentially performed as defined in the claims, including: a first step including colliding and mixing the primary or secondary alcohol compound, the dialkyl sulfoxide having 2 to 8 carbon atoms, and the acid anhydride having 4 to 10 carbon atoms in a flow-through type reactor to cause a reaction; and a second step including colliding and mixing, in a flow-through type reactor, the trialkylamine having 3 to 10 carbon atoms and the mixture liquid obtained in the first step to cause a reaction.

The first step may contain, in the flow-through type reactor, (3) a method including colliding and mixing the acid anhydride having 4 to 10 carbon atoms, and a liquid containing the primary or secondary alcohol compound and the dialkyl sulfoxide having 2 to 8 carbon atoms.

In the present invention, the method (3) is preferred.

### <Reactor>

In the present invention, at least the first and second steps are sequentially performed in a flow-through type reactor.

The flow-through type reactor may be of any type. Preferably, the flow-through type reactor is a microreactor.

The method (3) will be described as a typical example of the present invention. In the first step using the method (3), the mixing linear velocity is preferably 0.01 m/s to 6.0 m/s, more preferably 1.0 m/s to 6.0 m/s, further more preferably 1.1 m/s to 5.5 m/s, particularly preferably 1.2 m/s to 5.0 m/s.

In this regard, referring to Fig. 1, the mixing linear velocity corresponds to, for example, the linear velocity of the fluid immediately downstream of point 5 (the mixing start point in the first step) and the linear velocity of the fluid immediately downstream of point 8 (the mixing start point in the second step).

The reaction temperature in the first step is preferably from -30 to 30°C, more preferably from -20 to 20°C, further preferably from -15 to 15°C, and particularly preferably from -10 to 10°C.

The reaction time in the first step is preferably from 0.001 to 10 seconds, more preferably from 0.03 to 5 seconds, and further preferably from 0.05 to 3 seconds.

The mixing linear velocity in the second step is preferably from 0.5 m/s to 5.0 m/s, more preferably from 1.0 m/s to 4.5 m/s, and further preferably from 1.2 m/s to 4.0 m/s.

The reaction temperature in the second step is preferably from -30 to 50°C, more preferably from -20 to 50°C, further preferably from -15 to 45°C, and particularly preferably from -10 to 40°C.

The reaction time in the second step is preferably from 0.1 to 120 seconds, more preferably from 0.3 to 60 seconds, and further preferably from 0.5 to 30 seconds.

The flow passage of the micromixer (e.g., each flow passages between points 4 and 5, between points 5 and 6, and immediately downstream of point 5, shown in Fig. 1) may have any inner diameter or any cross-sectional area. In the present invention, the inner diameter (cross-sectional area) of the flow passage is preferably 0.05 to 5.0 mm (0.00196 to 19.6 mm²), more preferably 0.10 to 2.5 mm (0.00785 to 4.9 mm²), further more preferably 0.15 to 1.5 mm (0.018 to 1.8 mm²).

In this regard, the cross-sectional shape of the flow passage described above may be circular or square.

The microreactor has very small flow passages capable of mixing a plurality of liquids. If necessary, the microreactor may have feed passages that communicate with the flow passages to feed liquids to the flow passages. If necessary, the microreactor may further have components other than the flow passages and the feed passages.

The microreactor for use in the present invention is not particularly limited as long as it has very small flow passages capable of mixing a plurality of liquids. The microreactor may be appropriately selected, depending on the purpose, for example, from a micromixer (such as a substrate type micromixer or a joined pipe micromixer) and a branched tube.

The substrate type micromixer, which is sometimes called a microchannel, is composed of a substrate and flow passages formed inside the substrate or on the surface of the substrate.

The substrate type micromixer may be of any type as long as the effect of the present invention is not impaired. The substrate type micromixer may be appropriately selected, depending on the purpose, from, for example, the mixer described in WO 96/30113 A having very small flow passages for mixing; and the mixer described in W. Ehrfeld, V. Hessel, H. Lowe, "Microreactors," Chapter 3, published by Wiley-VCH.

Besides the flow passages described above, the substrate type micromixer preferably has feed passages that communicate the flow passages to feed a plurality of liquids to the flow passages. In other words, the micromixer preferably has a structure in which the upstream side of the flow passage described above is branched depending on the number of feed passages.

The number of feed passages is not particularly limited, and can be appropriately selected depending on the purpose. Preferably, a plurality of liquids to be mixed are fed from separate feed passages and joined and mixed together in the flow passage. Alternatively, the micromixer may be so configured that one liquid can be previously charged into a flow passage, and another liquid can be fed from a feed passage to the flow passage.

The joined pipe micromixer has flow passages formed in the interior and optionally connection means for connecting tubes to the flow passages formed in the interior. The connection means is not particularly limited, and can be appropriately selected depending on the purpose from known tube connection types, such as a screw type, a union type, a butt weld type, a socket welding type, a socket weld type, a flange type, a bite type, a flare type, and a mechanical type.

Besides the flow passages described above, feed passages that communicate the flow passages to feed a plurality of liquids to the flow passages are preferably formed in the interior of the joined pipe micromixer. In other words, the micromixer preferably has a structure in which the upstream side of the flow passage described above is branched depending on the number of feed passages. When two feed passages are provided, the joined pipe micromixer may be of, for example, T-shaped or Y-shaped. When three feed passages are provided, the joined pipe micromixer may be, for example, cruciform. Alternatively, the micromixer may be so configured that one liquid can be previously charged into a flow passage, and another liquid can be fed from a feed passage to the flow passage.

The material of the micromixer is not particularly limited, and can be appropriately selected depending on requirements such as heat resistance, pressure resistance, solvent resistance, and easy workability. Examples of the material of the micromixer include metals or alloys including stainless steel, titanium, copper, nickel, aluminum, and Hastelloy (registered trademark), silicone, fluororesins such as Teflon (registered trademark) and PFA (perfluoroalkoxy resin), and TFAA (trifluoroacetamide).

The micromixer is configured to accurately control the flow of the reactant solution by means of its microstructure. Therefore, the micromixer is preferably manufactured by micro-machining technology.

Any micro-machining technology is not particularly limited, and can be appropriately selected depending on the purpose, for example, from (a) LIGA technology using X-ray lithography in combination with electroplating, (b) high aspect ratio photolithography using EPON-SU8, (c) mechanical micro-cutting (e.g., micro-drill processing with a high speed rotatable drill with a diameter of the order of a micrometer), (d) high aspect ratio processing of silicon using deep RIE, (e) hot embossing, (f) optical shaping, (g) laser processing, and (h) ion beam techniques.

The micromixer may be any of commercially available micromixers such as a microreactor having an interdigital channel structure, a single mixer and a caterpillar mixer manufactured by Institut für Mikrotechnik Mainz GmbH (IMM), Micro Glass Reactor manufactured by Mikroglas GmbH, CYTOS manufactured by CPC Systems GmbH, YM-1 Mixer and YM-2 Mixer manufactured by Yamatake Co., Ltd., Mixing Tee and Tee (T-shaped connector) manufactured by SHIMADZU GLC Ltd., IMT Chip Reactor manufactured by Institute of Microchemical Technology Co., Ltd., Micro Hi-Mixer developed by Toray Engineering Co., Ltd., Union Tee manufactured by Swagelok Company, a mixer manufactured by YMC CO., LTD., and a mixer manufactured by Nakamura Choukou Co., Ltd.

Any of these micromixers may be used alone as a microreactor, or a tube reactor may be connected to the downstream part of any of these micromixers to form an extended flow passage. The length of the flow passage can be adjusted by connecting a tube reactor to the downstream part of the micromixer.

The time of retention of the mixed liquid (reaction time) is proportional to the length of the flow passage.

In this regard, the tube reactor is a reactor for accurately controlling the time required for solution to undergo reaction (retention time control) after the solution is prepared by quick mixing by the micromixer.

The tube reactor is not particularly limited. For example, the inner diameter, outer diameter, length, material, and other features of the tube may be appropriately selected depending on the desired reaction.

The tube reactor may be any of commercially available products, such as a stainless steel tube manufactured by GL Sciences Inc. (outer diameter 1/16 inches (1.58 mm), inner diameter selectable from 250 µm, 500 µm, 800 µm, and 1,000 µm, 1/8 inches (3.17 mm), with the tube length adjustable by the user).

The material of the tube reactor is not limited, and any of the above examples of the material of the micromixer is preferably used to form the tube reactor.

The flow passage described above has the functions of mixing a plurality of liquids by diffusion and removing heat of reaction.

The method of mixing the liquids in the flow passage described above is not particularly limited, and can be appropriately selected depending on the purpose, for example, from laminar mixing and turbulent mixing. In particular, laminar mixing (static mixing) is preferred because it allows more efficient reaction control or heat removal. Since the flow passage of the microreactor is very small, a plurality of liquids fed from the feed passages tends to spontaneously form dominantly laminar flows so that they are diffused and mixed in directions perpendicular to the flows. For laminar mixing, branch and meeting points may be provided in the flow passage to divide the laminar flow cross-section of the liquid, so that the mixing rate can be increased.

Turbulent mixing (dynamic mixing) may be provided in the flow passage of the microreactor. In this case, laminar flow can be changed to turbulent flow by controlling the flow rate and the geometry of the flow passage (such as the three-dimensional shape of the liquid contact portion, the bending of the flow passage, or other shapes, or the wall surface roughness). Turbulent mixing has advantage in that it can provide higher mixing efficiency and higher mixing rate than laminar mixing.

In this regard, the inner diameter, the cross-sectional area, length, and cross-sectional shape of the flow passage are not particularly limited and can be appropriately selected depending on the purpose.

While the flow passage of the micromixer has been described above, the flow passage of the reactor preferably has a cross-sectional area of 0.00196 to 19.6 mm². The cross-section may have any shape as long as it has such a cross-sectional area.

The feed passages have the functions of communicating with the flow passage described above and feeding a plurality of liquids to the flow passage. In general, other sides of the feed passages than their sides communicating with the flow passage described above are connected to containers containing the liquids to be mixed.

The feed passages may have any inner diameter as long as the effect of the present invention is not impaired. The inner diameter of the feed passages can be appropriately selected depending on the purpose.

When the microreactor has a plurality of feed passages, they may have the same or different inner diameters.

Components other than the flow passages and the feed passages described above are not particularly limited and can be appropriately selected depending on the purpose. Such components include, for example, a pump for use in liquid feeding, temperature control means, reaction acceleration means, a sensor, and a tank for storing the produced compound.

The pump is not particularly limited, and can be appropriately selected from industrially useful pumps. Pumps free of pulsation during liquid feeding are preferred, such as plunger pumps, gear pumps, rotary pumps, and diaphragm pumps.

The temperature control means is not particularly limited and can be appropriately selected depending on the reaction temperature. Examples of the temperature control means include a thermostatic chamber, a circulator, a heat exchanger, and a backpressure valve.

The reaction acceleration means can be appropriately selected depending on the liquids to be mixed or the desired reaction. Examples of the reaction acceleration means include vibrational energy applying means, heating means, light irradiation means, and voltage applying means. The microreactor having voltage applying means may be, for example, the micro-flow electrochemical reactor disclosed in JP-A-2006-104538.

The sensor is not particularly limited, and examples thereof include a temperature sensor, a flow rate sensor, and a pressure sensor for measuring the pressure in the flow passage.

A plurality of raw materials may be supplied at the same or different flow rates to the microreactor. The pump for use in liquid feeding may be any industrially useful liquid feed pump. Preferably, the pump is of a type that can be as free as possible of pulsation during liquid feeding. The pump is preferably a plunger pump, a gear pump, a rotary pump, a diaphragm pump or the like.

In the microreactor, liquid or solution-state compounds are mixed and reacted by the kinetic energy of the flowing liquid and solution. If necessary, however, energy for accelerating mixing, such as vibrational energy, may be applied from outside the microreactor. The mixing can be changed from static mixing (laminar flow) to dynamic mixing (turbulent flow) according to the flow rate and the geometry of the reactor (such as the three-dimensional shape of the liquid contact portion, the bending of the flow passage, or other shapes, or the wall surface roughness). The mixing may be any of turbulent mixing and laminar mixing.

Hereinafter, a preferred reactor according to the present invention will be described with reference to Fig. 1.

Fig. 1 is a schematic configuration diagram showing a preferred production system according to the present invention. In this case, the reaction step shown in Fig. 1 is based on Swern oxidation.

Hereinafter, Fig. 1 will be briefly described.

In Fig. 1, solution A is a solution containing an alcohol compound and a sulfoxide compound, and solution B is a solution containing an acid anhydride, and solution C is a solution containing a base.

Points 1, 2 and 3 each are a port for supplying a raw material to the microreactor. Points 4, 6, 7 and 9 each are a port for supplying a raw material to the micromixer. Point 5 is a mixing start point in a first step (a reaction between solutions A and B). Point 8 is a mixing start point in a second step (a reaction with solution C). Point 10 is the exit of the microreactor.

In this case, section 1 to 4 (a section between points 1 and 4) is a section for controlling the temperature of solution A. Section 2 to 6 (a section between points 2 and 6) is a section for controlling the temperature of solution B. Section 3 to 9 (a section between points 3 and 9) is a section for controlling the temperature of solution C. Moreover, section 5 to 7 (a section between points 5 and 7) is a section for the reaction in the first step. Section 8 to 10 (a section between points 8 and 10) is a section for the reaction in the second step.

The aldehyde compound or the ketone compound obtained by the production method of the present invention can be isolated by a known method. For example, the aldehyde compound or the ketone compound is isolated and purified using, as needed, one or any appropriate combination of extraction with an organic solvent, distillation, reprecipitation with an organic solvent, water, or a mixture of an organic solvent and water, crystallization, and column chromatography.

### <Use of aldehyde compound and ketone compound>

The aldehyde compound or the ketone compound produced by the production method of the present invention is useful not only as an industrial product or an intermediate of an industrial product but also as an antibiotic or a synthetic intermediate thereof in the case of a 9- or more-membered ring macrolide compound (preferably a compound of any of the ring structures A to I above).

### EXAMPLES

The present invention will be described in more detail based on examples given below.

The structure of the synthesized product was identified by NMR, IR, and milli-mass spectroscopy.

The reaction rate to the desired product was determined by analyzing the content from the peak area ratio using high performance liquid chromatography (HPLC).

The HPLC measurement was performed under the following conditions: column, Waters XBridge C18; eluent, acetonitrile/water (V/V = 7/3); buffer, 0.1% phosphoric acid; detector, UV 210 to 254 nm Corona CAD or RI; flow rate, 1.0 mL/min; column temperature, 30 to 40°C.

The isolation yield of the product for which no standard material was commercially available was determined by silica gel column chromatography.

### Example 1

The reaction was performed using the reactor shown in Fig. 1.

The solutions used were a methylene chloride solution of a mixture of macrolide alcohol compound 1 (0.06 M) and dimethyl sulfoxide (0.15 M) with the adjusted concentrations (solution A), a trifluoroacetic anhydride/methylene chloride solution (0.15 M) (solution B), a diisopropylethylamine/methylene chloride solution (0.18 M) (solution C).

Pump LC-20AT or LC-10Ai manufactured by SHIMADZU CORPORATION was used to feed the solutions A, B and C to the microreactor. The solutions A, B and C were supplied at a flow rate of 1.5 mL/min to the microreactor. In this process, the retention times at sections 5 to 7, and 8 to 10 of Fig. 1 were 0.05 seconds and 6.9 seconds, respectively. Moreover, the reaction temperature in the first step was 0°C, and the reaction temperature in the second step was 30°C. In addition, the mixture linear velocity in the first step was 1.6 m/s (the diameter of the flow passage: 0.2 mm, the flow passage sectional area: 0.03 mm²).

After a waiting time of 3 minutes, the reaction liquid flowing out of the exit of the reactor was sampled for 15 seconds into a sampling tube containing 1 mL of pure water and then stirred at 25°C for 10 seconds. The reaction solution was analyzed using HPLC, in which quantitative analysis was performed by internal standardization using a standard material. As a result, the total yield of the desired ketone compound was 96%.

### Comparative Example 1

Solutions A, B, and C were used, which were adjusted for concentration in the same method as in Example 1. Beforehand, 4.5 mL of the solution A was added to a 25 mL flask containing a magnetic stirrer bar. After the solution A was cooled to 0°C, 4.5 mL of the solution B was added at a rate of 1.5 mL/min dropwise to the flask with stirring. After the dropwise addition was completed, the mixture was stirred for 10 minute. While maintaining the reaction liquid at 0°C, subsequently, 4.5 mL of the solution C was added at a rate of 1.5 mL/min dropwise to the flask with stirring. After the dropwise addition was completed, the reaction liquid was raised to 30°C, and the mixture was stirred for 10 minute. The reaction solution after the reaction for 10 minutes was analyzed using HPLC, in which quantitative analysis was performed by internal standardization using a standard material. As a result, the yield of the desired ketone compound was 10%, the oxidation reaction was accompanied by a side reaction with the substrate, so that the yield was significantly lowered.

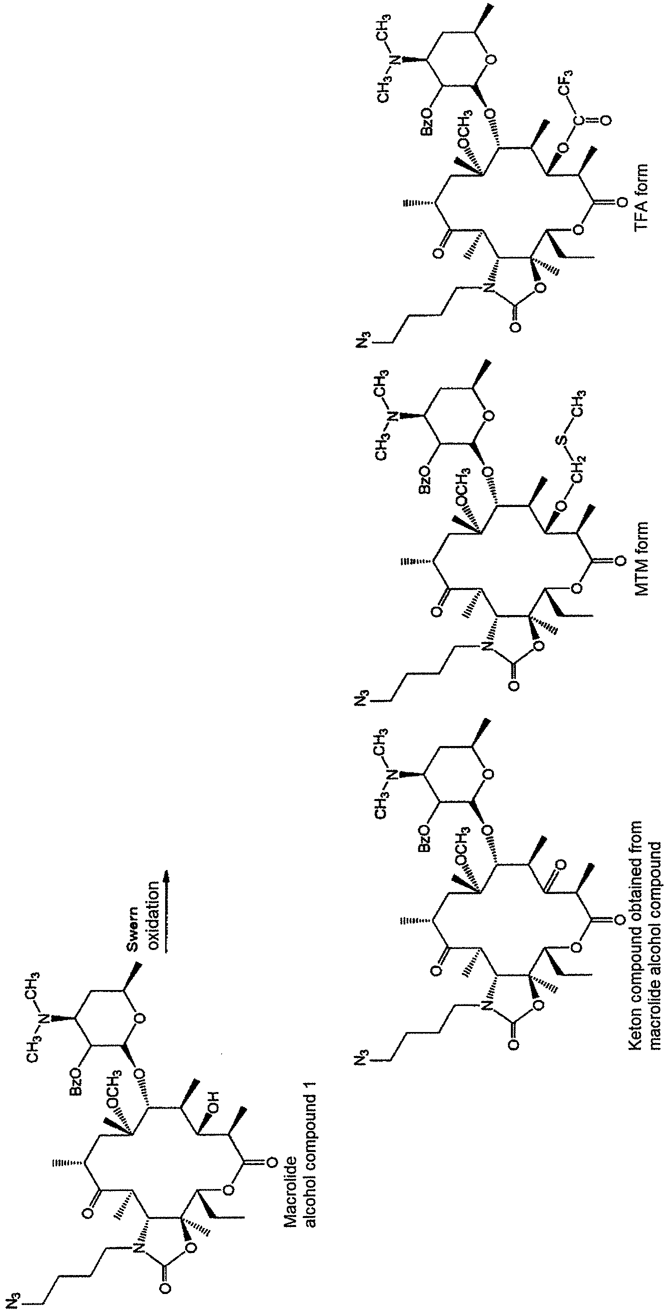

Herein, "Bz" represents a benzoyl group [-C(=O)-C₆H₅].

The obtained results are comprehensively shown in the following Table 1.

In this regard, the MTM form is a product derived from the raw material alcohol compound by converting -OH to -O-CH₂-S-CH₃, and the TFA form is a product derived from the raw material alcohol compound by converting -OH to -O-C(=O)-CF₃.

The impurities are side reaction products other than the Pummerer rearrangement products.

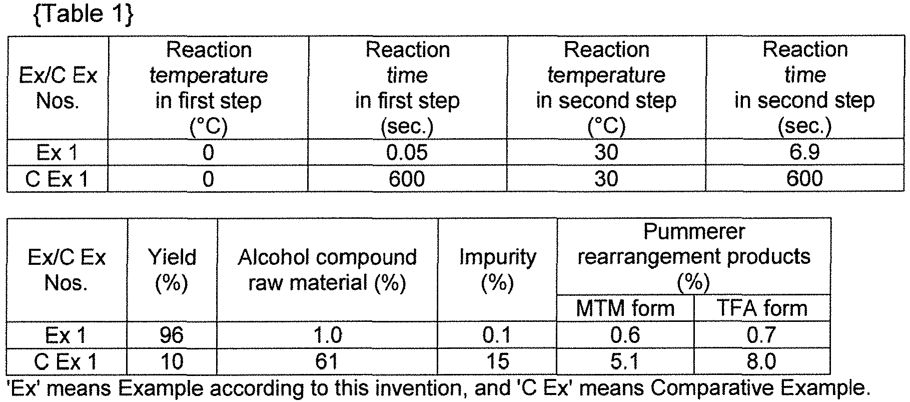

### Examples 2 to 7

Examples 2 to 7 were performed under the same conditions as those in Example 1, except that the first step was performed under the reaction temperature conditions shown in Table 2 below.

The obtained results are comprehensively shown in the following Table 2.

**{Table 2}**

| Ex Nos. | Reaction temperature in first step (°C) | Yield (%) | Alcohol compound raw material (%) | Impurity (%) | Pummerer rearrangement products (%) | |
|---|---|---|---|---|---|---|
| | | | | | MTM form | TFA form |
| Ex 2 | -30 | 85 | 12 | N.D. | 0.1 | 0.4 |
| Ex 3 | -20 | 94 | 2.1 | 0.1 | 0.3 | 0.6 |
| Ex 4 | -10 | 95 | 1.5 | 0.1 | 0.4 | 0.6 |
| Ex 5 | 10 | 94 | 0.9 | 0.2 | 0.4 | 0.8 |
| Ex 6 | 20 | 92 | 0.8 | 0.2 | 0.5 | 1.1 |
| Ex 7 | 30 | 58 | 18 | 7.1 | 5.2 | 11 |

| | | | | | | |
|---|---|---|---|---|---|---|
| 'Ex' means Example according to this invention. | | | | | | |

### Examples 8 to 12

Examples 8 to 12 were performed under the same conditions as those in Example 1, except that the second step was performed under the reaction temperature conditions shown in Table 3 below

The obtained results are comprehensively shown in the following Table 3.

In the tables, N.D. means that the impurities were not detected or at a level lower than the detection limit.

**{Table 3}**

| Ex Nos. | Reaction temperature in second step (°C) | Yield (%) | Alcohol compound raw material (%) | Impurity (%) | Pummerer rearrangement products (%) | |
|---|---|---|---|---|---|---|
| | | | | | MTM form | TFA form |
| Ex 8 | -30 | 71 | 23 | N.D. | 0.1 | 0.2 |
| Ex 9 | -20 | 88 | 3.4 | 0.2 | 0.2 | 0.4 |
| Ex 10 | -10 | 90 | 3.0 | 0.2 | 0.3 | 0.4 |
| Ex 11 | 40 | 96 | 0.9 | 0.3 | 0.6 | 0.9 |
| Ex 12 | 50 | 94 | 2.4 | 0.6 | 0.8 | 1.3 |

| | | | | | | |
|---|---|---|---|---|---|---|
| 'Ex' means Example according to this invention. | | | | | | |

### Comparative Example 13 and Examples 14 to 16

Comparative Example 13 and Examples 14 to 16 were performed under the same conditions as those in Example 1, except that the first step was performed under the mixture linear velocity conditions shown in Table 4 below.

The obtained results are comprehensively shown in the following Table 4.

In the table, the flow passage diameter means the inner diameter of the micromixer, and the flow passage sectional area means the area of the liquid flowing cross-section of the flow passage.

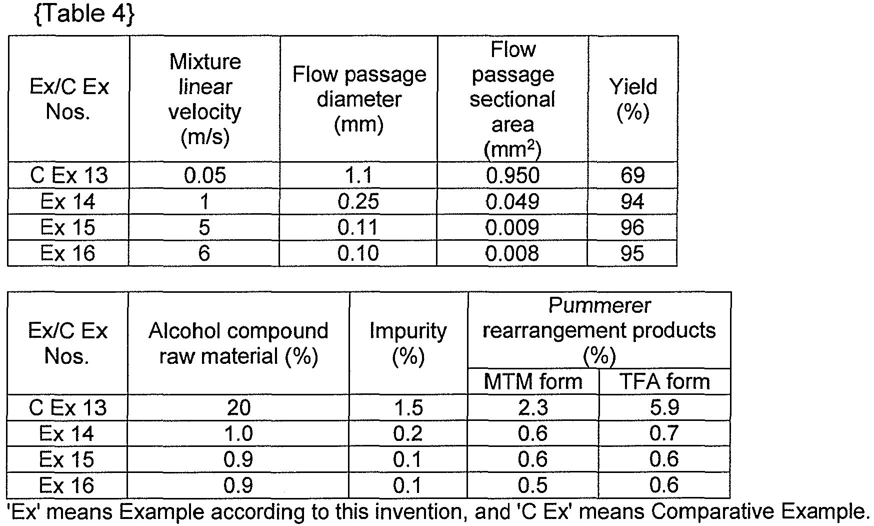

### Examples 17 to 20

Examples 17 to 20 were performed under the same conditions as those in Example 1, except that the alcohol compounds shown in Table 5 below were used instead.

The obtained results are comprehensively shown in the following Table 5.

**{Table 5}**

| Ex Nos. | Alcohol compound raw material | Yield (%) | Raw material (%) | Impurity (%) | Pummerer rearrangement products (%) | |
|---|---|---|---|---|---|---|
| | | | | | MTM form | TFA form |
| Ex 17 | 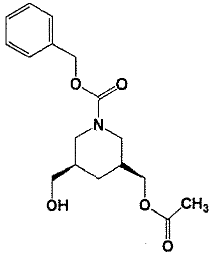 | 93 | 1.1 | 0.2 | 0.3 | 0.4 |
| Ex 18 | 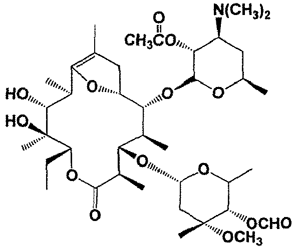 | 89 | 1.2 | 0.8 | 0.9 | 1.2 |
| Ex 19 | 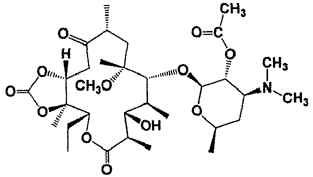 | 84 | 1.6 | 0.7 | 0.8 | 0.7 |
| Ex 20 | 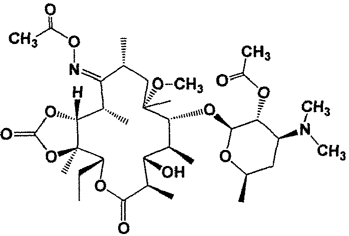 | 82 | 2.1 | 1.1 | 1.1 | 0.9 |

| | | | | | | |
|---|---|---|---|---|---|---|
| 'Ex' means Example according to this invention. | | | | | | |

The results in Tables 1 to 5 show that the production method of the present invention makes it possible to obtain a desired aldehyde compound or ketone compound with high yield and high purity by highly selective oxidation reaction of a primary or secondary alcohol compound having a nucleophilic substituent or a substituent vulnerable to nucleophilic attack.

In Comparative Examples 1 and 13, the amount of the Pummerer rearrangement products was relatively large, and the amount of impurities, which may be generated due to involvement of a nucleophilic substituent in the reaction or involvement of a substituent vulnerable to nucleophilic attack in the reaction, was as large as 15%. In contrast, according the present invention, even in the presence of a nucleophilic substituent or a substituent vulnerable to nucleophilic attack, only the target -OH can be converted to a carbonyl group with substantially no involvement of such a substituent in the reaction.

### REFERENCE SIGNS LIST

- 1, 2, 3: Port for supplying a raw material to the microreactor
- 4, 6, 7, 9: Port for supplying a raw material to the micromixer
- 5: Mixing start point in a first step (a reaction between solutions A and B)
- 8: Mixing start point in a second step (a reaction with solution C)
- 10: Exit of the microreactor
- 5: Mixing start point in a first step (a reaction between solutions A and B)
- 8: Mixing start point in a second step (a reaction with solution C)
- 10: Exit of the microreactor

## Claims

1. A method of producing an aldehyde compound or a ketone compound by oxidation of a primary or secondary alcohol compound having, in the molecule, a group selected from an amino group, an azido group, a hydroxy group, and a hydroxy group protected with a protective group, the method comprising the steps of:
a first step including colliding and mixing an acid anhydride having 4 to 10 carbon atoms and a liquid containing the alcohol compound and a dialkyl sulfoxide having 2 to 8 carbon atoms in a flow-through type reactor to cause a reaction; and
a second step including colliding and mixing, in a flow-through type reactor, a trialkylamine having 3 to 10 carbon atoms and the mixture liquid obtained in the first step to cause a reaction;
wherein the reactions in the first step and the second step are sequentially performed, and a mixture linear velocity in the first step is in a range of from 1 m/s to 6 m/s.

2. The production method according to Claim 1, wherein the aldehyde compound or the ketone compound has a group selected from the amino group, the azido group, the hydroxy group, and the hydroxy group protected with a protective group, which are derived from the molecule of the alcohol compound.

3. The production method according to Claim 1 or 2, wherein the hydroxyl group protected with a protective group is an acyloxy group, a sulfonyloxy group, a sulfonylamino carbonyloxy group, a silyloxy group, an alkoxy ethyleneoxy group, an alkylthio ethyleneoxy group, or a group represented by Formula (p) described below: 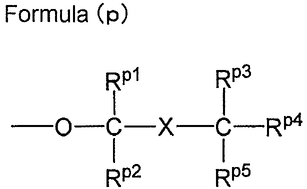 wherein X represents -O- or -S-; R^{p1} to R^{p5} each independently represent a hydrogen atom, an alkyl group, or an aryl group; and R^{p1}, and R^{p2} or R^{p3} may combine together to form a ring.

4. The production method according to any one of Claims 1 to 3, wherein the alcohol compound has, in its molecule, at least two groups selected from an amino group, an azido group, a hydroxy group, and a hydroxy group protected with a protective group.

5. The production method according to any one of Claims 1 to 4, wherein the alcohol compound is a cyclic alcohol compound.

6. The production method according to any one of Claims 1 to 5, wherein the alcohol compound is a cyclic alcohol compound having -C(=O)-, -O-C(=O)- or -C(=O)-O- as ring-constituting atoms or bonds.

7. The production method according to any one of Claims 1 to 6, wherein the alcohol compound is a cyclic alcohol compound having a 6- or more-membered ring.

8. The production method according to any one of Claims 1 to 7, wherein the alcohol compound has two or more asymmetric carbon atoms.

9. The production method according to any one of Claims 1 to 8, wherein the reaction temperature in the first step is from -30 to 30°C.

10. The production method according to any one of Claims 1 to 9, wherein the reaction temperature in the second step is from -30 to 50°C.

## Patentansprüche

1. Verfahren zur Herstellung einer Aldehydverbindung oder einer Ketonverbindung durch Oxidation einer primären oder sekundären Alkoholverbindung, die im Molekül eine Gruppe aufweist, ausgewählt aus einer Aminogruppe, einer Azidogruppe, einer Hydroxygruppe und einer Hydroxygruppe mit einer Schutzgruppe, wobei das Verfahren die Schritte umfasst:
einen ersten Schritt, umfassend das Kollidieren lassen und Mischen eines Säureanhydrids mit 4 bis 10 Kohlenstoffatomen und einer Flüssigkeit, die die Alkoholverbindung enthält, und eines Dialkylsulfoxids mit 2 bis 8 Kohlenstoffatomen in einem Durchflussreaktor, um eine Reaktion zu bewirken; und
einen zweiten Schritt, einschließlich das Kollidieren lassen und Mischen in einem Durchflussreaktor, eines Trialkylamins mit 3 bis 10 Kohlenstoffatomen und der Mischflüssigkeit, die in dem ersten Schritt erhalten wurde, um eine Reaktion zu bewirken;
wobei die Reaktionen im ersten Schritt und im zweiten Schritt nacheinander durchgeführt werden und eine lineare Mischgeschwindigkeit im ersten Schritt in einem Bereich von 1 m/s bis 6 m/s liegt.

2. Herstellungsverfahren nach Anspruch 1, wobei die Aldehydverbindung oder die Ketonverbindung eine Gruppe aufweist, ausgewählt aus der Aminogruppe, der Azidogruppe, der Hydroxygruppe und der Hydroxygruppe, die mit einer Schutzgruppe geschützt ist, die von dem Molekül der Alkoholverbindung abgeleitet sind.

3. Herstellungsverfahren nach Anspruch 1 oder 2, wobei die mit einer Schutzgruppe geschützte Hydroxylgruppe eine Acyloxygruppe, eine Sulfonyloxygruppe, eine Sulfonylaminocarbonyloxygruppe, eine Silyloxygruppe, eine Alkoxyethylenoxygruppe, eine Alkylthioethylenoxygruppe, oder eine Gruppe ist, dargestellt durch die nachstehend beschriebene Formel (p): 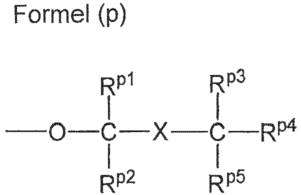 worin X für -O- oder -S- steht; R^{p1} bis R^{p5} unabhängig voneinander ein Wasserstoffatom, eine Alkylgruppe oder eine Arylgruppe darstellen; und R^{p1} und R^{p2} oder R^{p3} sich zu einem Ring verbinden können.

4. Herstellungsverfahren nach einem der Ansprüche 1 bis 3, wobei die Alkoholverbindung in ihrem Molekül mindestens zwei Gruppen enthält, ausgewählt aus einer Aminogruppe, einer Azidogruppe, einer Hydroxygruppe und einer Hydroxygruppe, die mit einer Schutzgruppe geschützt ist.

5. Herstellungsverfahren nach einem der Ansprüche 1 bis 4, wobei die Alkoholverbindung eine zyklische Alkoholverbindung ist.

6. Herstellungsverfahren nach einem der Ansprüche 1 bis 5, wobei die Alkoholverbindung eine cyclische Alkoholverbindung mit -C(=O)-, -OC(=O)- oder -C(=O)-O- als Ring konstituierende Atome oder Bindungen aufweist.

7. Herstellungsverfahren nach einem der Ansprüche 1 bis 6, wobei die Alkoholverbindung eine cyclische Alkoholverbindung mit einem 6- oder mehrgliedrigen Ring ist.

8. Herstellungsverfahren nach einem der Ansprüche 1 bis 7, wobei die Alkoholverbindung zwei oder mehr asymmetrische Kohlenstoffatome aufweist.

9. Herstellungsverfahren nach einem der Ansprüche 1 bis 8, wobei die Reaktionstemperatur in dem ersten Schritt von -30 bis 30 °C beträgt.

10. Herstellungsverfahren nach einem der Ansprüche 1 bis 9, wobei die Reaktionstemperatur im zweiten Schritt von -30 bis 50 °C beträgt.

## Revendications

1. Procédé de production d' un composé aldéhyde ou d'un composé cétone par oxydation d'un composé alcool primaire ou secondaire ayant, dans la molécule, un groupe choisi parmi un groupe amino, un groupe azido, un groupe hydroxy, et un groupe hydroxy protégé par un groupe protecteur, le procédé comprenant les étapes de:
une première étape comprenant la collision et le mélange d'un anhydride d'acide ayant 4 à 10 atomes de carbone et d'un liquide contenant le composé alcoolique et un dialkyl sulfoxyde ayant 2 à 8 atomes de carbone dans un réacteur de type à écoulement continu pour provoquer une réaction; et
une deuxième étape comprenant la collision et le mélange, dans un réacteur de type à écoulement continu, d'une trialkylamine ayant 3 à 10 atomes de carbone et du mélange liquide obtenu dans la première étape pour provoquer une réaction;
dans lequel les réactions de la première étape et de la deuxième étape sont exécutées séquentiellement, et une vitesse linéaire du mélange dans la première étape est dans une plage de 1 m/s à 6 m/s.

2. Procédé de production selon la revendication 1, dans lequel le composé aldéhyde ou le composé cétone a un groupe choisi parmi le groupe amino, le groupe azido, le groupe hydroxy et le groupe hydroxy protégé par un groupe protecteur, qui sont dérivés de la molécule du composé alcoolique.

3. Procédé de production selon la revendication 1 ou 2, dans lequel le groupe hydroxyle protégé par un groupe protecteur est un groupe acyloxy, un groupe sulfonyloxy, un groupe sulfonylamino carbonyloxy, un groupe silyloxy, un groupe alcoxy éthylèneoxy, un groupe alkylthio éthylèneoxy, ou un groupe représenté par la formule (p) décrite ci-dessous: 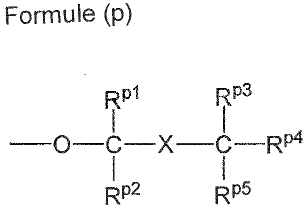 où X représente -O- ou -S-; R^{p1} à R^{P5} chaque indépendamment représentente un atome d'hydrogène, un groupe alkyle ou un groupe aryle; et R^{p1} et R^{p2} ou R^{p3} peuvent se combiner pour former un cycle.

4. Procédé de production selon l'une quelconque des revendications 1 à 3, dans lequel le composé d'alcool a, dans sa molécule, au moins deux groupes choisis parmi un groupe amino, un groupe azido, un groupe hydroxy, et un groupe hydroxy protégé par un groupe protecteur.

5. Procédé de production selon l'une quelconque des revendications 1 à 4, dans lequel le composé alcool est un composé d'alcool cyclique.

6. Procédé de production selon l'une quelconque des revendications 1 à 5, dans lequel le composé alcool est un compose d'alcool cyclique ayant -C(=O)-, -OC(= O)- ou -C(=O)-O- comme atomes ou liaisons constituant le cycle.

7. Procédé de production selon l'une quelconque des revendications 1 à 6, dans lequel le composé d'alcool est un composé d'alcool cyclique ayant un cycle à 6 chaînons ou plus.

8. Procédé de production selon l'une quelconque des revendications 1 à 7, dans lequel le composé alcoolique a deux ou plusieurs atomes de carbone asymétriques.

9. Procédé de production selon l'une quelconque des revendications 1 à 8, dans lequel la température de réaction dans la première étape est de -30 à 30 °C.

10. Procédé de production selon l'une quelconque des revendications 1 à 9, dans lequel la température de réaction dans la deuxième étape est de -30 à 50 °C.
